# EUROPÄISCHE PATENTSCHRIFT

(11) **EP 2 253 335 B1**
(45) Veröffentlichungstag und Bekanntmachung des Hinweises auf die Patenterteilung: **04.09.2013**
(21) Anmeldenummer: 10075187.4
(22) Anmeldetag: 06.05.2010
(51) Int. Cl.: A61L 11/00

(54) **Verfahren und Vorrichtung zur Keimabtötung in Abfällen**
Method and device for killing of germs in waste
Procédé et dispositif destinés à la suppression des germes dans des déchets

(30) Priorität: 07.05.2009 DE 102009021054
(43) Veröffentlichungstag der Anmeldung: 24.11.2010
(73) Patentinhaber: Dünnebeil, Andreas, 14513 Teltow (DE)
(72) Erfinder: Dünnebeil, Andreas, 14513 Teltow (DE)

(56) Entgegenhaltungen:
- WO-A1-03/024633
- WO-A2-2008/081028
- US-A- 5 397 480
- US-A- 5 424 046

## Beschreibung

Die Erfindung betrifft ein Verfahren und eine Vorrichtung zur Durchführung des Verfahrens zur Keimabtötung in Abfällen, insbesondere bei in Rohrleitungen förderfähigen Reststoffen für und von Biogasanlagen, die bei einer Temperatur oberhalb von 100°C sterilisiert werden müssen.

Substrate, die in Biogasanlagen stofflich umgesetzt und deren Reststoffe anschließend hygienisch einwandfrei weitergegeben werden sollen, müssen häufig, um den gesetzlichen Anforderungen zu entsprechen, sterilisiert bzw. hygienisiert werden. Dabei wird gemäß den gesetzlichen Bestimmungen unterschieden zwischen der Hygienisierung, meist bei 70°C für eine Stunde, und der Sterilisation, meist bei 133°C für 20 Minuten.
Biogasanlagen setzen organische Substrate in verwertbare Biogase (wie Methan) um. Dieses Gase dienen meist zur Strom- und Wärmegewinnung vor Ort. Neuerdings werden derartige Gase nach einer Aufbereitung auch in das Gasnetz eingespeist. Die Reststoffe der Biogasanlagen werden meist in der Landwirtschaft verwertet. Einige der auf dem Markt verfügbaren Substrate wie z.B. Schlachtabfälle, müssen aber hygienisiert bzw. sterilisiert werden, wenn die Reststoffe der Biogasanlage landwirtschaftlich verwertet werden sollen.
Hygienisierungsanlagen bzw. Sterilisationsanlagen müssen grundsätzlich so aufgebaut sein, dass sichergestellt wird, dass die Substrate auch die vorgegebene Zeit unter der erforderlichen Temperatur verweilen. Dieses wird üblicherweise durch einen Batchbetrieb erreicht. Dabei wird eine Charge aufgeheizt und verweilt anschließend die erforderliche Zeit in einem Reaktor. Nach Verstreichen der Zeit wird die Charge abgelassen und die nächste Charge in gleicher Weise behandelt. Je nach Erfordernissen muss die abfließende Charge abgekühlt werden. Anlagen bestehen meist aus ein, zwei oder drei Reaktoren.
Das Aufheizen der Substrate erfolgt meist über Wärmetauscher oder eine Dampfinjektion. Bei Wärmetauschern kommen Heizwasser und Thermoöl zum Einsatz. Häufig wird die Wärme aus dem Bereich des Blockheizkraftwerkes gewonnen, dass zur Verstromung des Biogases dient. Die Abkühlung erfolgt ebenfalls über Wärmetauscher oder eine Flash-Verdampfung. Bei der Flash-Verdampfung ist das Substrat in Bezug auf den zugehörigen Druck soweit überhitzt, dass es zu einer spontanen Verdampfung kommt, bis durch den Enthalpieverlust über das Ausdampfen das Substrat soweit abgekühlt ist, dass der Dampfdruck etwa dem Umgebungsdruck des Substrates entspricht.

Das Substrat muss ggf. vor der wärmetechnischen Behandlung zerkleinert werden. Hierfür sind sehr viele apparatetechnische Lösungen auf dem Markt, die für das Verfahren aber nicht von grundsätzlicher Bedeutung sind. Es muss sichergestellt sein, dass das Substrat mit entsprechenden Pumpen z.B. Excenterschneckenpumpen, förderfähig ist und ggf. gesetzlichen Anforderungen entspricht.

So beschreibt die DE 41 11 253 ein Verfahren zur Entseuchung und Hygienisierung von Abfall und/oder Wertstoffen aus der Tierkadaververwertung, bei der die Entkeimung mittels überhitzten Dampfes erfolgt. Der Nachteil des Verfahrens ist, das beim gesetzlich vorgeschriebenen Batchbetrieb ein kontinuierlicher bzw. quasikontinuierlicher Substratdurchsatz in der Anlage ohne Durchmischung der Substratchargen nicht möglich ist.

In der DE 42 00 588 ist zwar eine kontinuierliche Heißsterilisierung pumpfähigen Behandlungsgutes mittels einer sich drehenden Rohrwendel beschrieben, in dem beschriebenen Verfahren ist jedoch als Nachteil des Verfahrens ein Batchbetrieb nicht möglich.

Bei dem US Patent 5,424,046 handelt es sich bei der Vorrichtung um eine Sterilisiervorrichtung für feste Abfälle bei der gemäß Fig. 7 aus US Patent 5,424,046 die Drainage ( Flüssige Phase) separat abgeführt wird. Der Reaktor hat eine ähnliche Zielsetzung, erreicht dieses aber durch einen grundsätzlich anderen Aufbau. So werden feste Abfälle in dem Reaktor behandelt und ein Durchmischen/Rühren erfolgt nicht. Gleichzeitig kann eine flüssige Phase (Kondensate) abgeführt werden.

Bei unserem Verfahren werden alle Inhaltsstoffe des Reaktors gemeinsam aus dem Reaktor abgeführt. Vorteilig ist die gemeinsame Abfuhr aus dem Reaktor dadurch, dass der flüssige Substratstrom in den dann größeren Mengen sich besser zur Wärmerückgewinnung nutzen lässt. Weiterhin unterscheidet sich unser Verfahren durch die nach außen kontinuierliche Betriebsweise, wodurch eine im Gegensatz zum Stand der Technik wesentlich geringe Heizleistungsspitze erforderlich ist. Dadurch kann die gesamte Anlage energetisch und apparativ günstiger gestaltet werden. Zu Spalte 10 Z 63 aus US Patent 5,424,046. Hier wird beschrieben, dass das Wasser aus dem Dampf als Kondensat abgeführt wird.

In unserer Erfindung erfolgt eine gemeinsame Verwertung des gesamten Substratstroms zur optimalen Wärmenutzung. Dazu ist es von Vorteil, wenn das Substrat einen möglichst geringen Feststoffgehalt aufweist, um den Wärmeübergang zu optimieren. Eine separate Kondensatabscheidung, die einzeln auch noch behandelt werden müsste wäre hier eher nachteilig. Zu Spalte 11 Zeile 10 aus US Patent 5,424,046 gilt gleiches, da wir kein separates Kondensat erzeugen wollen.

Zu Spalte 8 Zeile 50:Die beiden Prozesse unterscheiden sich in grundlegender Art wie zuvor beschrieben. Sie zeichnen sich aber, wie eine Vielzahl technischer Prozesse der allgemeinen Verfahrenstechnik, durch einen wiederkehrenden Kreislauf aus. Was aus unserer Sicht keine Erfinderhöhenschädlichkeit nach sich zieht.

Bei WO 03/024633 A1 handelt es sich um ein Verfahren für feste Abfälle. Im Gegensatz dazu beschreibt die hier vorliegende Erfindung ein Verfahren zur Behandlung von flüssigen, pumpfähigen Substraten/Abfällen. Dieses zeigt sich beispielsweise auch durch die eingesetzten Rührwerke. Zusätzlich kann bei unserem Verfahren kein separates Kondensat entstehen wie in Figur 1 aus WO 03/024633 A1 dargestellt.

In S. 17 Absatz 6 aus WO 03/024633 A1 ist ein kontinuierlicher Prozess erwähnt aber nicht detailliert dessen Umsetzung dargestellte. Bei der Erfindung WO 03/024633 A1 handelt es sich aber um eine Behandlung von festen Abfällen mit eine expliziten Kondensatabscheidung.

Bei WO 2008/081028 A2 handelt es sich um ein Verfahren zur Behandlung fester Abfälle du Substrate. Bei unserem Verfahren handelt es sich um flüssige Substrate, deren weiterführende Trocknung in den in WO 2008/081028 A2 aufgeführten Trocknertypen nicht vorgesehen und meist nicht sinnvoll ist, da die Substrate einem ganz anderen Verwendungszweck, nämlich der Biogaserzeugung zugeführt werden sollen.

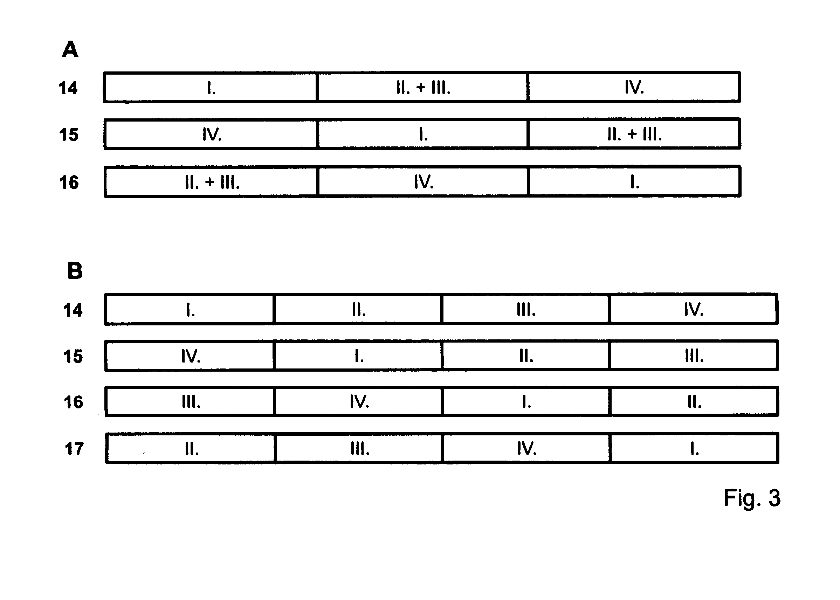

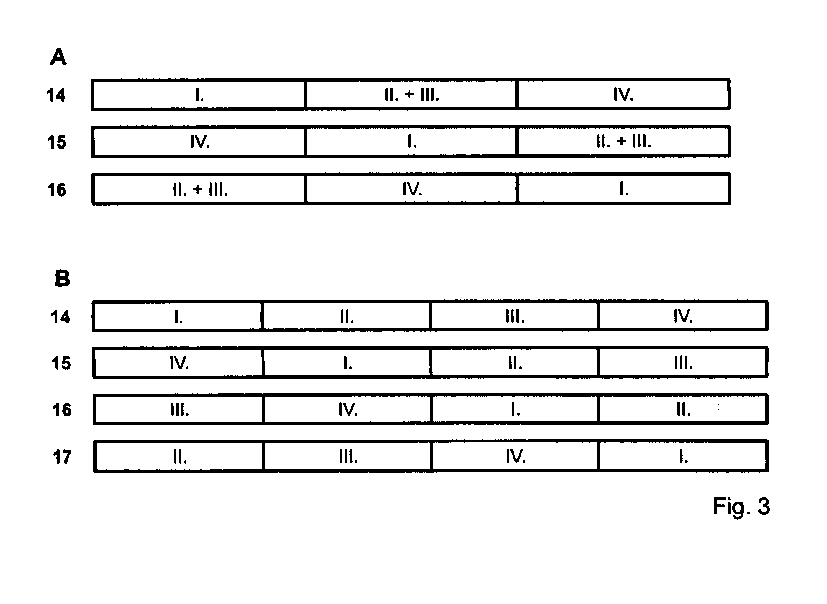

In Figur 3 ist ein Kühlprozess beschrieben bei dem im geschlossenen Kreislauf aus dem Reaktor über den Wärmetauscher 45' die Wärme abgeführt wird. Das vorgekühlte Substart wird dann nach Abschluss der Kühlung im Reaktor über den Heizwassererzeuger weiter gekühlt. Gegenüber dieser in den einzelnen Reaktoren batchweisen Kühlung arbeitet unsere Erfindung im Bereich der Kühlung kontinuierlich mit dem Gesamtsubstratstrom. Damit kann die in den Substraten enthaltene Wärme in für den jeweiligen Einsatzzweck angepassten Aggregaten maximal genutzt werden.

Aufgabe der Erfindung ist somit, das Verfahren Hygienisierung bzw. Sterilisation ohne Durchmischung der einzelnen Chargen im Batchbetrieb soweit wie möglich kontinuierlich zu betreiben, um periphere Ressourcen wie z.B. Energie oder Kühlwasser möglichtst gleichmäßig zu nutzen und keine kostenintensiven Spitzenleistungen abzufordern. Dabei soll auf Wärmetauscher, deren Oberfläche mit dem Substrat in Berührung kommt, verzichtet werden können.

Erfindungsgemäß werden diese Aufgaben durch das Verfahren entsprechend der Merkmale des Anspruchs 1 und durch die Vorrichtung entsprechend der Merkmale des Anspruchs 10 gelöst.

Vorteilhafte Ausführungen der Erfindung sind in den kennzeichnenden Merkmalen der Unteransprüche beschrieben.

Hauptkennzeihen der Erfindung ist dabei, dass das Substrat abwechselnd in mindestens vier Reaktoren gepumpt wird, wodurch sich die größtmögliche Kontinuität für den Substratfluss ergibt. Der Verfahrensablauf ist dabei in vier gleichlange Zeitintervalle gegliedert. Das erste Zeitintervall umfasst die Substratzufuhr in den ersten Reaktor, die Aufheizzeit in dem zweiten Reaktor, die Haltezeit für die Temperatur im dritten Reaktor und den Ablauf aus dem vierten Reaktor. Im zweiten Zeitintervall wird analog dazu im ersten Reaktor aufgeheizt, im zweiten die Temperatur gehalten, im dritten das Substrat abgeführt und im vierten zugeführt, etc., wodurch ein kontinuierlicher Verfahrens-prozess ermöglicht wird, ohne das Material der einzelnen Chargen mischen zu müssen. Alternativ können auch drei Reaktoren parallel betrieben werden. Es werden dann die Aufheizzeit und die Haltezeit in einem Zeitintervall zusammengefasst. Die Länge der Zeitintervalle richtet sich nach dem Intervall, das die längste Zeit erfordert. Dadurch ist die Kontinuität der Wärmeabnahme / des Wärmebedarfs aber nicht mehr ganz gegeben. Alle Zeitintervalle in einem System sind jeweils gleich lang. Zu- und Ablauf aus den Reaktoren sind aber zusammen gesehen kontinuierlich.

Im Reaktor wird dem Substrat zur Erhitzung Dampf über Dampflanzen zugeführt. Die Dampflanzen sind über Führungsrohre, die an der Behälterwand befestigt sind, geführt. Am Behälterumfang sind mehrere Dampflanzen vorgesehen. Das Substrat wird zumindest während der Dampfzufuhr kontinuierlich durch ein Behälterrührwerk umgewälzt. Durch die laufende Erneuerung des Substrats an der Dampflanzenspitze wird verhindert, dass an der Spitze einer jeden Dampflanze sich ein Dampfpolster aufbaut. Die befestigten Dampflanzenführungen dienen gleichzeitig als Strombrecher im Reaktor, um eine Trombenströmung zu verhindern. Die Dampflanzen können in unterschiedliche Höhen enden.

Der Ablauf aus den Reaktoren erfolgt über eine Pumpe in einen Flash-Tank. Der Pumpe nachgeschaltet ist direkt vor dem Flash-Tank eine Druckhaltearmatur. So wird eine Dampfblasenbildung innerhalb der Rohrleitung verhindert. Die Pumpe ist als Verdrängerpumpe ausgebildet und sorgt damit gleichzeitig für einen kontinuierlichen Substratstrom zum Flash-Tank hin. In dem Flash-Tank verdampft in Abhängigkeit vom herrschenden Druck ein Teil der Wasserphase des Substrats; herrscht in dem Flash-Tank beispielsweise ein Druck von ca. 70 hPa absolut, liegt die Verdampfungstemperatur bei etwa 40 °C. Die Energie wird über diesen Dampf aus dem Substrat entfernt. Das im Flash-Tank abgekühlte Substrat wird über eine Pumpe aus dem System entfernt. Das System der Rückkühlung über einen Flash-Tank ist besonders für größere Substratströme geeignet. Bei kleineren Substratströmen kann auch ein Flächenwärmetauscher eingesetzt werden, in dem über einen Kühlwasserstrom der Substratstrom direkt gekühlt wird.

Die Energie, die in dem Abdampf, den Brüden, aus dem Flash-Tank enthalten ist, muss aus dem System entfernt werden. In einem Kondensator, vorzugsweise ein Sprühkondensator, werden die Dämpfe niedergeschlagen. Im oberen Teil ist dafür eine Düse vorgesehen, die das Kühlmedium, im Kreis gefahrenes Kondensat, in feine Tröpfchen zerstäubt und gleichmäßig über den Behälterquerschnitt verteilt. Die Tröpfchen erzeugen eine große Oberfläche, an denen die Brüden auskondensieren. Im unteren Teil des Kondensators sammelt sich das Kondensat aus den Tröpfchen und wird über eine Pumpe durch einen Wärmetauscher abgekühlt wieder zurück zur Düse gefördert. Durch die kontinuierliche Kondensation reichert sich das Kondensat im System an. Der Überschuss wird so aus dem System entfernt, dass der Füllgrad im Kondensator konstant bleibt.

Ergebnis ist somit, dass mit der vorliegenden Erfindung in einem kontinuierlichen Prozess ohne Durchmischung der einzelnen Chargen Abfälle wie Schlachtabfälle und Reststoffe für und aus Biogasanlagen im Batchbetrieb sterilisiert bzw. hygienisiert werden können, wobei im Gegensatz zum bisherigen Stand der Technik durch den kontinuierlichen Prozess keine Energie- und Kühlwasserspitzenleistungen mehr abgefordert werden müssen und die gesamte Anlage somit bei gleichem Stoffumsatz per Zeiteinheit sehr viel kleiner und somit kostenextensiver ausgelegt werden kann. Ein weiterer Vorteil der Erfindung ist, dass auf Wärmetauscher verzichtet werden kann.

Somit werden die der Erfindung zugrunde liegenden Aufgaben durch das bereitgestellte Verfahren sowie durch die bereitgestellte Vorrichtung zur Durchführung des Verfahrens vollkommen gelöst.

Nachfolgend wird ein bevorzugtes Ausführungsbeispiel der Erfindung weiter unter Bezugnahme auf die beigefügten Zeichnungen erläutert. Es zeigen:

**Fig.1** eine schematische Darstellung der Erfindung in Form eines Fließbildes, **Fig.2** einen vertikalen Schnitt durch einen Reaktor in zwei Ausführungen und **Fig.3** den Verlauf der Zeitintervalle in den einzelnen Reaktoren zueinander.

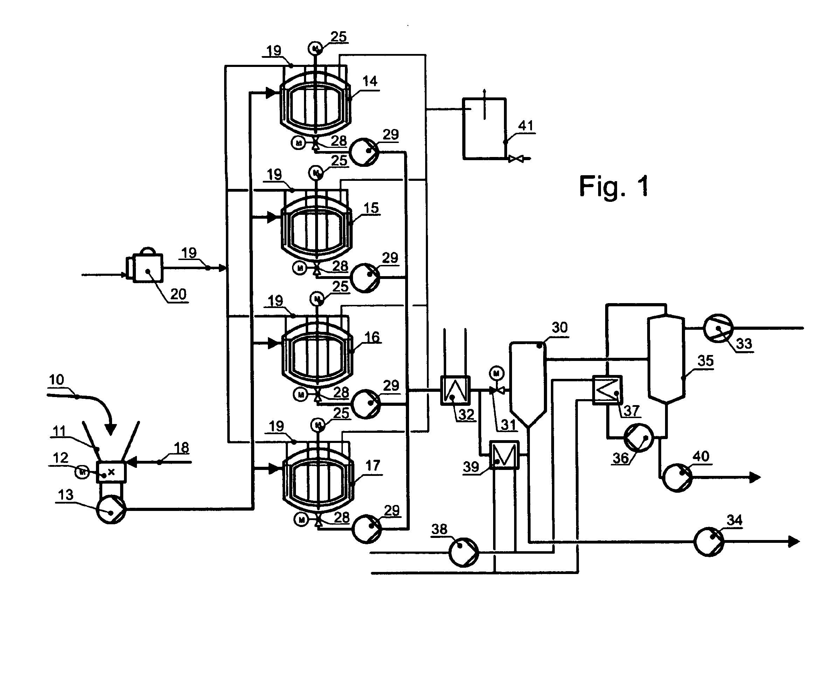

**Fig.1** zeigt den grundsätzlichen Aufbau des Verfahrens mit vier Reaktoren. Das Substrat (10), hier als Beispiel Schlachtabfälle die für 20 Minuten bei 133 °C sterilisiert werden müssen, wird über einen Trichter (11) einem Zerkleinerungsaggregat (12) zugeführt. Das zerkleinerte Substrat wird anschließend über eine Pumpe (13) den Reaktoren (14 bis 17) zugeführt. Weist das Substrat eine zu hohe Festigkeit bzw. einen zu hohen Feststoffgehalt auf, kann in dem Bereich des Zerkleinerungsaggregats ggf. noch Maischwasser (18) zur Verdünnung hinzugefügt werden. Mit höherem Feststoffgehalt werden die Substrate allgemein zunehmend schlechter pumpfähig. Daher kann es in gewissen Fällen durchaus sinnvoll sein, die Substrate zu verdünnen, um deren Pumpfähigkeit sicher zu stellen. Da bei dem Verfahren aber grundsätzlich keine Wärmetauscher am Substrat eingesetzt werden müssen, sind die hydraulischen Eigenschaften der Substrate aber nicht von so primärer Bedeutung wie bei Verfahren mit Flächenwärmetauschern.

Nachdem der jeweilige Reaktor (14 bis 17) bis auf sein Nennvolumen gefüllt ist, wird das Substrat in dem Reaktor im nächsten Zeitintervall mit Dampf (19) aufgeheizt. Der Dampf wird über den Dampfkessel (20) erzeugt. Neben einem Dampfkessel, der mit fossilen Brennstoffen oder mit Biogas beheizt wird, kann auch ein Abhitzekessel verwandt werden, der seine Wärme z.B. aus den Rauchgasen eines Blockheizkraftwerks bezieht.

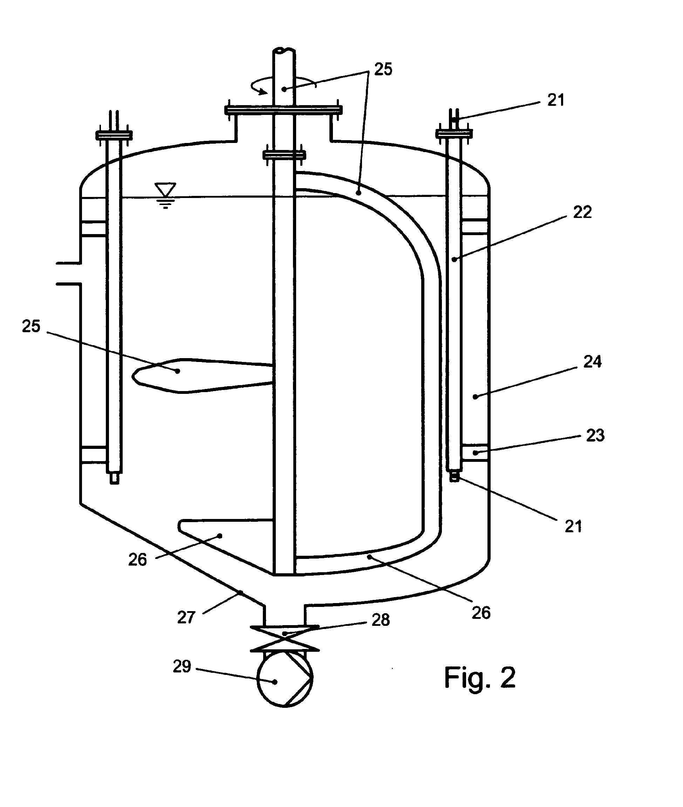

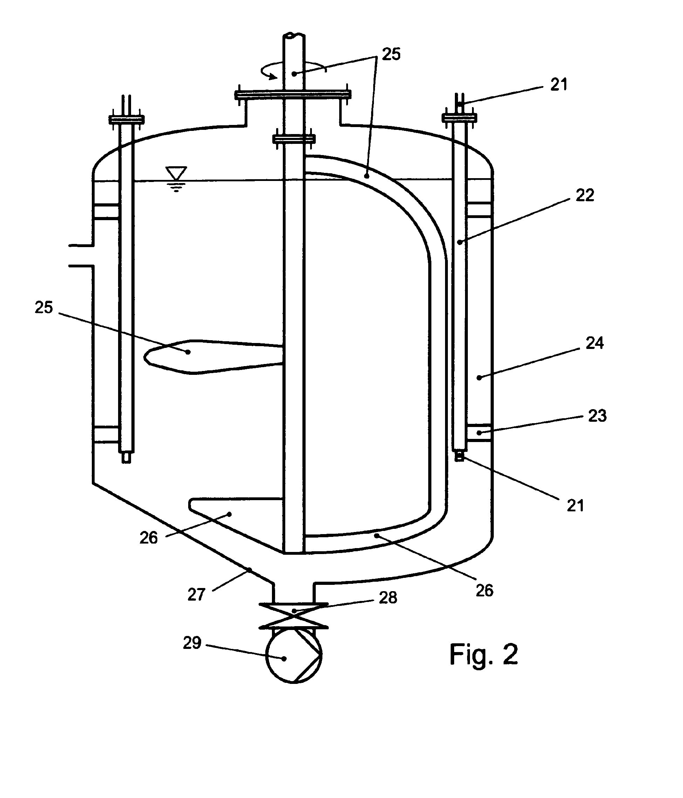

Der Dampf wird in den jeweiligen Reaktor (14 bis 17) (**Fig.2**), über Dampflanzen (21) eingebracht. Es werden mehrere Dampflanzen eingebracht, meist 2 bis 8, um eine bessere Verteilung der Dampfeinbringung zu erreichen. Die Dampflanzen sind am Umfang des Reaktors angebracht und werden über Führungsrohre (22) gehalten. Die Führungsrohre sind mit Rohren (23) und/oder Blechen (24) an der Reaktorwand befestigt und dienen gleichzeitig als Strombrecher. Möglich sind bei einem entsprechendem wandgängigen Rührer auch freitragende Dampflanzen die eher auf halben Radius des Behälters angeordnet sind. Während der Dampfeinbringung läuft das Rührwerk (25). Das Rührwerk kann nach Erfordernissen an die Durchmischung unterschiedlich ausgeführt sein. Es sollte aber mit einer Austragshilfe (26) versehen sein. Die Austragshilfe (26) kann entfallen, wenn ein ausreichend steiler Konusboden (27) vorgesehen ist, so dass das behandelte Substrat selbstständig aus dem Behälter abfließt. Am Ablauf des Reaktors ist ein Absperrorgan (28) vorgesehen. Direkt unterhalb dieses Absperrorgans (28) ist eine Pumpe (29) vorgesehen. Die Pumpe dient in erster Linie einer gewissen Druckerhöhung im abfließenden Substrat, um Dampfschläge in der Leitung bis zu dem Flash-Tank (30) zu minimieren. Wird die Pumpe als Verdrängerpumpe, z.B. als Excenterschneckenpumpe, ausgeführt, kann die Pumpe gleichzeitig zur Dosierung des abfließenden Substrats verwandt werden. Direkt vor dem Flash-Tank (30) ist eine Druckhaltearmatur (31) vorgesehen, um den Druck in der Gesamtleitung zu halten. Wenn es die Stückigkeit des Substrates zulässt, reicht an Stelle der Druckhaltearmatur auch eine feste Düse am Eintritt in den Flash-Tank (30).

Direkt nach dem Reaktor ist das abfließende Substrat durch die erhöhte Temperatur recht dünnflüssig und eignet sich für eine Wärmegewinnung über einen Wärmetauscher (32). Aus dem Substrat mit einer Temperatur von knapp oberhalb von 133 °C kann so z.B. 90 grädiges Heizwasser gewonnen werden. Für den Wärmetauscher eignen sich Systeme die zumindest auf einer Seite eine Schlammphase vertragen, denkbar ist beispielsweise ein Doppelrohrwärmetauscher.

Beim Eintritt in den Flash-Tank (30) verdampft ein Teil der im Substrat befindlichen Wasserphase entsprechend dem herrschenden Druck im Flash-Tank. Zur besseren Verteilung des Substrats über den Querschnitt kann der Eintritt in den Flash-Tank als Düse ausgebildet sein. Im Flash-Tank wird bei einer gewünschten Abkühltemperatur unter 100 °C mit einer Vakuumpumpe (33) ein Unterdruck erzeugt, so dass die zugehörige Sattdampftemperatur der gewünschten Ablauftemperatur des Substrates entspricht. Bei einer gewünschten Ablauftemperatur von beispielsweise 40 °C muss somit ein Druck von ca. 70 hPa absolut in dem Flash-Tank vorliegen. Die abgekühlten Substrate sammeln sich im Bodenbereich des Flash-Tanks (30) und werden über die Pumpe (34) aus dem System abgeführt.

Die im Flash-Tank (30) frei werdenden Dämpfe sind meist noch mit einem gewissen Luft- / Gasanteil aus dem Substrat versetzt und werden als Brüden bezeichnet. Die Brüden werden im oberen Bereich aus dem Flash-Tank (30) abgezogen und in den Brüdenkondensator (35) über die Vakuumpumpe (33) gesaugt. Damit kein Substrat aus dem Flash-Tank (30) in den Bereich der Kondensation gelangt, kann der Brüdenabzug der Flash-Tanks über einen Demister geschützt sein.

Der Brüdenkondensator (35) ist vorzugsweise als Sprühkondensator konzipiert. Im oberen Bereich ist ein Düsenkopf angeordnet, der kühle Flüssigkeit in Tröpfchenform über den Querschnitt verteilt. Die Brüden werden von unten hinzugegeben und von der Vakuumpumpe (33) nach oben gesaugt. Dabei kondensieren die Wasseranteile weitestgehend aus. Das Kondensat sammelt sich im unteren Bereich des Brüdenkondensators und wird über einen Kreislauf mit der Zirkulationspumpe (36) wieder zum Düsenkopf gefördert. Im Zirkulationskreis wird das Kondensat im Wärmetauscher (37) soweit abgekühlt, dass es wiederum zur Kondensation genutzt werden kann. Die Wärme wird abschließend über Kühlwasser mit einer Pumpe (38) abgeführt. Überschüssiges Kondensat wird über die Pumpe (40) abgeführt.

Bei kleineren Substratströmen kann die Rückkühlung auch aus wirtschaftlichen Gründen über einen Flächenwärmetauscher (39) erfolgen.

Die Reaktoren (14 bis 17) stehen gerade beim Betrieb als Sterilisation bei einer Betriebstemperatur oberhalb von 100 °C unter Überdruck. Zur Sicherheit werden alle Reaktoren mit einer Berstscheibe ausgerüstet. Sollte der Fall eintreten, dass eine Berstscheibe bricht, muss das austretende Medium, Dampf mit Substrat, schadlos abgeleitet werden. Dieser Stoffstrom wird in dem Zirkulationsabscheider (41) von den Fest- und Flüssigstoffen befreit. Die Restgase und -dämpfe werden ins Freie abgeblasen.

Fig.3 zeigt den zeitlichen Ablauf für Anlagen Fall A mit drei Reaktoren (14 bis 16) bzw. Fall B mit vier Reaktoren (14 bis 17). Unterschieden wird zwischen den verfahrenstechnischen Aufgaben:
I. Füllen
II. Aufheizen
III. Hygienisieren / Sterilisieren (Behandlung)
IV. Leeren

Im Fall A (**Fig.3**) werden Aufheiz- und Behandlungszeit zusammengefasst. Alle drei Zeitintervalle sind etwa gleich lang. Im Fall B wird jede der verfahrenstechnischen Aufgaben in einem eigenen, gleichlangen Zeitintervall vollzogen.

Die Gleichheit der Zeitintervalle kann geringfügig durch Umschaltzeiten zwischen den einzelnen verfahrenstechnischen Aufgaben abweichen.

### Bezugszeichenliste

- **10**: Substrat
- **11**: Trichter
- **12**: Zerkleinerungsaggregat
- **13**: Pumpe
- **14**: Reaktor 1
- **15**: Reaktor 2
- **16**: Reaktor 3
- **17**: Reaktor 4
- **18**: Maischwasser
- **19**: Dampf
- **20**: Dampfkessel
- **21**: Dampflanze
- **22**: Führungsrohr
- **23**: Rohr
- **24**: Blech
- **25**: Rührwerk
- **26**: Austragshilfe
- **27**: Konus
- **28**: Absperrarmatur
- **29**: Pumpe
- **30**: Flash-Tank
- **31**: Druckhaltearmatur
- **32**: Wärmetauscher
- **33**: Vakuumpumpe
- **34**: Pumpe
- **35**: Brüdenkondensator
- **36**: Zirkulationspumpe
- **37**: Wärmetauscher
- **38**: Pumpe
- **39**: Flächenwärmetauscher
- **40**: Pumpe
- **41**: Zirkulationsabscheider

## Patentansprüche

1. Verfahren zur Keimabtötung in Abfällen wobei ohne Durchmischung der einzelnen Substratchargen zwischen den Reaktoren zeitlich versetzt alle notwendigen Verfahrensschritte nacheinander durchgeführt werden, wobei das von Keimen zu befreiende Substrat dem Verfahrensprozess kontinuierlich zugeführt wird, wobei
a) das Substrat abwechselnd in mindestens vier Reaktoren (Fig. 1, 14-17) eingefüllt wird,
b) das Substrat mittels Dampfinjektion (19) abwechselnd in diesen Reaktoren aufgeheizt und somit behandelt, d.h. von Keimen befreit wird,
c) das Substrat anschließend abwechselnd kontinuierlich aus diesen Reaktoren abgeführt wird, und
d) das Substrat anschließend kontinuierlich abgekühlt (32, 30) wird.

2. Verfahren nach Anspruch 1, **dadurch gekennzeichnet, dass** bei Hygienisierung die Aufheizung des Substrats bei Temperaturen zwischen 50°C und 100°C über eine Zeit von 30 Minuten und 24 Stunden erfolgt und bei Sterilisierung die Aufheizung des Substrats bei Temperaturen zwischen 100°C und 150°C über eine Zeit von 10 bis 60 Minuten erfolgt.

3. Verfahren nach Anspruch 1, **dadurch gekennzeichnet, dass** das nach der Behandlung d.h. Entkeimung abwechselnd aus den Reaktoren abgeführte Substrat mittels einer geeigneten Abkühlvorrichtung (32, 30) abgekühlt wird.

4. Verfahren nach Anspruch 1, **dadurch gekennzeichnet, dass** zur Ermöglichung eines kontinuierlichen Verfahrensprozesses die vier Reaktoren (14 bis 17) eingesetzt werden, wobei diese jeweils abwechselnd den verfahrenstechnischen Aufgaben
a) Füllen,
b) Aufheizen,
c) Behandeln und
d) Leeren
zugeordnet sind, d.h. in jedem Reaktor ohne Durchmischung der einzelnen Substratchargen zeitlich versetzt alle diese Verfahrensschritte kontinuierlich nacheinander durchgeführt werden.

5. Verfahren nach Anspruch 4, **dadurch gekennzeichnet, dass** zur Ermöglichung eines kontinuierlichen Verfahrensprozesses die Zeitintervalle der verfahrenstechnischen Aufgaben jeweils etwa gleich groß sind.

6. Verfahren nach Anspruch 1, **dadurch gekennzeichnet, dass** die Abkühlung des Substrates in einem Flash-Verdampfer (30) in einem Druckbereich zwischen 30 und 1013 hPa absolut erfolgt.

7. Verfahren nach Anspruch 1, **dadurch gekennzeichnet, dass** zur Substratabkühlung ein zweistufiges Flash-Verdampfersystem mit Brüdenkondensation (35) verwendet wird, um aus dem ersten Flash-Verdampfersystem Wärme in Form von Heizwasser zu gewinnen (37).

8. Verfahren nach Anspruch 1, **dadurch gekennzeichnet, dass** eine Wärmerückgewinnung der Wärme aus dem Ablauf der Anlage zum Zulauf der Anlage über die Dampfphase erfolgt.

9. Verfahren nach Anspruch 6, **dadurch gekennzeichnet, dass** die Wärmeenergie in Form von Dampf aus dem Flash-Verdampfer in einer Brüdenkondensation (35) niedergeschlagen wird.

10. Vorrichtung zur Keimabtötung in Abfällen wobei ohne Durchmischung der einzelnen Substratchargen in jedem Reaktor zeitlich versetzt alle notwendigen Verfahrensschritte d.h. Füllen, Aufheizen, Behandeln und Leeren der Reaktoren, nacheinander durchgeführt werden können, umfassend
a) mindestens vier Reaktoren (14 - 17), in denen abwechselnd das von Keimen zu befreiende Substrat eingefüllt und erhitzt wird,
b) mindestens eine Dampfinjektionsvorrichtung (Fig. 2, 22) in diesen Reaktoren zur Erhitzung des von Keimen zu befreienden Substrats,
c) mindestens eine Abkühlvorrichtung (Fig. 1, 32) des abwechselnd aus den Reaktoren abgeführten Substrats.

11. Vorrichtung nach Anspruch 10, **dadurch gekennzeichnet, dass** sie mit Reaktoren (14 bis 17) mit von oben in Führungsrohren (22) eingebrachten Dampflanzen (21), einem Rührwerk (25) und anschließendem Flash-Tank (30) mit zugehöriger Brüdenkondensation (35), ausgestattet ist, und dass das Rührwerk mit einer geeigneten Austragshilfe versehen ist.

12. Vorrichtung nach Anspruch 10, **dadurch gekennzeichnet, dass** der Austrag des Substrats selbsttätig durch einen ausreichend steilen Konus (27) sichergestellt ist.

## Claims

1. Process for the destruction of germs in waste whereby, without the mixing action of the individual substrate lots between the reactors, all necessary process steps are carried out consecutively and time-delayed, whereby the substrate which is to be freed from germs is continuously fed into the process, whereby
a) the substrate is alternately filled into at least four reactors (Fig. 1, 14-17),
b) the substrate is heated alternately in these reactors using steam injection (19) and thus treated, i.e. is freed from germs,
c) the substrate is subsequently alternately and continuously discharged from these reactors, and
d) the substrate is subsequently continuously cooled down (32, 30).

2. Process according to claim 1, **distinguished by the fact** that with hygienization the substrate is heated at temperatures between 50°C and 100°C over a period of 30 minutes and 24 hours and with sterilisation the substrate is heated at temperatures between 100°C and 150°C over a period of 10 to 60 minutes.

3. Process according to claim 1, **distinguished by the fact** that the substrate alternatively discharged from the reactors after the treatment i.e. removal of germs is cooled down by means of a suitable cooling-down unit (32, 30).

4. Process according to claim 1, distinguished by the fact that the four reactors (14 to 17) are used in order to enable a continuous process, whereby these are respectively alternately allocated to the technical process tasks
a) Filling,
b) Heating,
c) Treatment and
d) Emptying
i.e. in each reactor without a mixing action of the individual substrate lots all of these process steps are continuously carried out consecutively and time-delayed.

5. Process according to claim 4, **distinguished by the fact** that the time intervals of the technical process tasks are respectively approximately the same size in order to enable a continuous process.

6. Process according to claim 1, **distinguished by the fac**t that the cooling down of the substrate is carried out absolute in a flash vaporizer (30) in a pressure range between 30 and 1013 hPa.

7. Process according to claim 1, **distinguished by the fact** that a two-stage flash vaporizer system with vapour condenser (35) is used to cool the substrate down in order to extract heat from the first flash vaporizer system in the form of heating water (37).

8. Process according to claim 1, **distinguished by the fact** that a heat recovery of the heat from the outlet of the plant to the intake of the plant is carried out through the vaporization phase.

9. Process according to claim 6, **distinguished by the fact** that the heat energy is condensed in the form of steam from the flash vaporizer in a vapor condenser (35).

10. Device for the destruction of germs in waste whereby without a mixing action of the individual substrate lots in each reactor all necessary process steps i.e. filling, heating, treatment and emptying of the reactors can be carried out consecutively and time-delayed, **comprising**
a) at least four reactors (14 - 17), in which alternately the substrate, which is to be freed from germs, is filled and heated,
b) at least one steam injection device (Fig. 2, 22) in these reactors in order to heat the substrate which is to be freed from germs,
c) at least one cooling-down unit (Fig. 1, 32) of the substrate, which is to be alternatively discharged from the reactors.

11. Device according to claim 10, distinguished by the fact that it is equipped with reactors (14 to 17) with steam lances (21) which are inserted from above in control tubes (22), an agitator (25) and connected flash tank (30) with associated vapour condenser (35) and that the agitator is fitted with a suitable discharge device.

12. Device according to claim 10, **distinguished by the fact** that the discharge of the substrate is ensured self-acting by a sufficiently steep cone (27).

## Revendications

1. Procédé destiné à la suppression des germes dans des déchets dans lequel toutes les étapes nécessaires à la réalisation du procédé entre les réacteurs sont exécutées, sans que les différentes charges de substrat aient été mélangées, les unes après les autres de manière décalée dans le temps, et dans lequel le substrat à désinfecter est amené continuellement dans le procédé, dans lequel
a) le remplissage de substrat est effectué par alternance dans au moins quatre réacteurs (Fig. 1, 14 à 17),
b) le chauffage du substrat est effectué par alternance au moyen de l'injection de vapeur (19) dans ces réacteurs, qui a pour effet de traiter le substrat respectivement de supprimer les germes,
c) le substrat est ensuite évacué par alternance et continuellement de ces réacteurs et
d) le substrat est ensuite refroidi continuellement (32, 30).

2. Procédé selon la revendication 1, **caractérisé en ce que** le chauffage du substrat pour une hygiénisation est effectué à des températures situées entre 50 °C et 100 °C pendant une durée de 30 minutes et de 24 heures et le chauffage du substrat pour une stérilisation est effectué à des températures situées entre 100 °C et 150 °C pendant une durée de 10 à 60 minutes.

3. Procédé selon la revendication 1, **caractérisé en ce que** le substrat soumis au traitement, c'est à dire à l'action germicide, est évacué par alternance des réacteurs au moyen d'un dispositif de refroidissement approprié (32, 30).

4. Procédé selon la revendication 1, **caractérisé en ce que** les quatre réacteurs (14 à 17) sont utilisés dans un procédé continuel et que les fonctions technologiques
a) de remplissage,
b) de chauffage,
c) de traitement et
d) de vidage
leur sont attribuées par alternance, c'est-à-dire que toutes ces étapes du procédé sont effectuées continuellement les unes après les autres de manière décalée dans le temps dans chaque réacteur, sans que les différentes charges de substrat aient été mélangées.

5. Procédé selon la revendication 4, **caractérisé en ce que** les intervalles de temps des fonctions technologiques s'étendent sur une durée approximativement identique, afin de permettre un procédé continuel.

6. Procédé selon la revendication 1, **caractérisé en ce que** le refroidissement du substrat est effectué dans un évaporateur flash (30) à une plage de pression située entre 30 et 1013 hPa absolus.

7. Procédé selon la revendication 1, **caractérisé en ce que le** refroidissement du substrat est effectué au moyen d'un système d'évaporation flash à condensation des buées à deux étages (35), afin de régénérer de la chaleur sous forme d'eau chaude du premier système d'évaporation flash (37).

8. Procédé selon la revendication 1, **caractérisé en ce que** la régénération de chaleur se produit à base de la chaleur sortant du dispositif vers l'admission du dispositif via la phase vapeur.

9. Procédé selon la revendication 6, **caractérisé en ce que** l'énergie calorifique est précipitée sous forme de vapeur de l'évaporateur flash dans une condensation des buées (35).

10. Dispositif destiné à la suppression des germes dans des déchets dans lequel toutes les étapes nécessaires à la réalisation du procédé, c'est-à-dire le remplissage, le chauffage, le traitement et le vidage, sont exécutées dans chaque réacteur les unes après les autres de manière décalée dans le temps, sans que les différentes charges de substrat aient été mélangées, **comprenant**
au moins quatre réacteurs (14 à 17) dans lesquels le substrat à désinfecter est rempli et chauffé par alternance,
b) au moins un dispositif d'injection de vapeur (Fig. 2, 22) monté dans ces réacteurs en vue du chauffage du substrat à désinfecter,
c) au moins un dispositif de refroidissement (Fig. 1, 32) du substrat évacué par alternance des réacteurs.

11. Dispositif selon revendication 10, caractérisé en ce qu**e** les réacteurs (14 à 17) sont équipés de lances de vapeur (21) intégrées par le haut dans des tubes de guidage (22), d'un agitateur (25) et d'un réservoir flash monté en aval (30) avec une condensation des buées (35) et que l'agitateur est équipé d'un dispositif d'évacuation auxiliaire approprié.

12. Dispositif selon revendication 10, **caractérisé en ce que** l'évacuation du substrat est effectuée automatiquement et assurée via un cône à angle obtus suffisant (27).
